# EUROPÄISCHE PATENTANMELDUNG

(11) **EP 1 726 563 A1**
(43) Veröffentlichungstag der Anmeldung: **29.11.2006**
(21) Anmeldenummer: 06009964.5
(22) Anmeldetag: 15.05.2006
(51) Int. Cl.: B67D 5/33, G01N 33/22, B67D 5/37, G01S 13/00

(54) **Verfahren zum Erfassen der Kraftstoffart von Kraftfahrzeugen an einer Tankstelle**

(30) Priorität: 23.05.2005 DE 102005023656
(71) Anmelder: FAFNIR GmbH, D-22765 Hamburg (DE)
(72) Erfinder: Willmer, Klaus, 21033 Hamburg (DE); Kunter, Stefan, 22337 Hamburg (DE); Maurer, Christian, Dr., 22547 Hamburg (DE); Schrittenlacher, Wolfgang, Dr., 21075 Hamburg (DE)
(74) Vertreter: UEXKÜLL & STOLBERG

(57) **Zusammenfassung**

Bei einem Verfahren zum Erfassen der Kraftstoffart von Kraftfahrzeugen an einer Tankstelle wird vor Beginn und/oder zu Beginn eines Betankungsvorgangs ein tür ein Dieselkraftstoff-Kraftfahrzeug und/oder ein Vergaserkraftstoff-Kraftfahrzeug charakteristisches Merkmal gemessen und bewertet. Dazu kann mittels einer Messeinrichtung (14), die im Bereich eines Zapfventils (4) und/oder einer zugehörigen Zapfanlage angeordnet ist, eine Messung einer für ein Dieselkraftstoff-Kraftfahrzeug charakteristischen Größe durchgeführt werden, und zwar vorzugsweise dann, wenn das Zapfventil (4) in einen Einfüllstutzen (6) eingeführt ist. Die Messergebnisse werden bewertet und vorzugsweise zum Steuern des Betankungsvorgangs verwendet. Bei einer Variante des Verfahrens wird die von der Zündanlage von Vergaserkraftstoff-Kraftfahrzeugen abgegebene elektromagnetische Strahlung verwendet.

## Beschreibung

Die Erfindung betrifft ein Verfahren zum Erfassen der Kraftstoffart von Kraftfahrzeugen an einer Tankstelle sowie eine Vorrichtung zur Durchführung eines derartigen Verfahrens.

Neuerdings werden sogenannte Hochleistungskraftstoffe angeboten, die den Kunden von Tankstellen noch nicht vertraut sind. Dies bedingt zunehmend eine Verwechslungsgefahr der Kraftstoffsorten.

So kommen sehr häufig Fehlbetankungen an Dieselkraftstoff-Personenkraftwagen vor, die versehentlich mit Vergaserkraftstoff betankt werden. Der umgekehrte Fall, dass ein Vergaser-Fahrzeug mit Dieselkraftstoff betankt wird, ist weniger häufig, da ein Zapfventil für Dieselkraftstoff wegen seines größeren Durchmessers nicht in einen Vergaserkraftstoff-Einfüllstutzen passt. Auch würde im letzteren Fall kein größerer Schaden entstehen, außer dass das Fahrzeug stehen bleibt und seine Kraftstoffanlage gereinigt werden muss.

Im ersteren Fall sind hingegen Schäden an Motor und Einspritzanlage die Regel, was hohe Reparaturkosten zur Folge hat. Bei Autoverleihfirmen ist der Anteil der Fehlbetankungen sehr hoch und damit ein gravierendes Problem. Dies ist auch für die Ölgesellschaften problematisch, weil diese Vorgänge imageschädigend sind.

Man könnte alle Dieselkraftstoff-Kraftfahrzeuge mit einem Transponder ausstatten, der mit Hilfe eines darauf abgestimmten Geräts an der Zapfsäule erkannt wird, so dass sich Fehlbetankungen vermeiden lassen. Dies würde jedoch einen Eingriff an allen Dieselkraftstoff-Kraftfahrzeugen erfordern und ist daher flächendeckend nur schwer durchzuführen und als Lösung des geschilderten Problems ungeeignet.

Es ist Aufgabe der Erfindung, eine praktisch realisierbare und zuverlässige Möglichkeit zu schaffen, um Fehlbetankungen von Dieselkraftstoff-Kraftfahrzeugen mit Vergaserkraftstoff zu vermeiden.

Diese Aufgabe wird gelöst durch ein Verfahren zum Erfassen der Kraftstoffart von Kraftfahrzeugen an einer Tankstelle mit den Merkmalen des Anspruchs 1 sowie eine Vorrichtung zur Durchführung eines derartigen Verfahrens mit den Merkmalen des Anspruchs 24. Vorteilhafte Ausgestaltungen der Erfindung ergeben sich aus den Unteransprüchen.

Bei dem erfindungsgemäßen Verfahren zum Erfassen der Kraftstoffart von Kraftfahrzeugen an einer Tankstelle wird vor Beginn und/oder zu Beginn eines Betankungsvorgangs ein für ein Dieselkraftstoff-Kraftfahrzeug und/oder ein Vergaserkraftstoff-Kraftfahrzeug charakteristisches Merkmal gemessen und bewertet. Das Verfahren erlaubt es also festzustellen, ob ein gerade an einer gegebenen Zapfanlage der Tankstelle vorgefahrenes und zu betankendes Kraftfahrzeug ein Dieselkraftstoff-Kraftfahrzeug oder ein Vergaserkraftstoff-Kraftfahrzeug ist, so dass Fehlbetankungen verhindert werden können, wenn die Kraftstoffart des Kraftfahrzeugs nicht zu der des gezogenen Zapfventils passt.

Bei bevorzugten Ausführungsformen des erfindungsgemäßen Verfahrens wird eine Messung einer für ein Dieselkraftstoff-Kraftfahrzeug charakteristischen Größe mittels einer Messeinrichtung durchgeführt, die im Bereich eines Zapfventils und/oder einer zugehörigen Zapfanlage angeordnet ist. Dabei können auch mehrere Einzelmessungen erfolgen. Vorzugsweise wird die Messung durchgeführt, wenn das Zapfventil in einen Einfüllstutzen eingeführt ist. Das Messergebnis wird bewertet.

Bei der Zapfanlage mit dem Zapfventil handelt es sich vorzugsweise um eine Zapfanlage für Vergaserkraftstoff, denn die oben erläuterten Probleme treten auf, wenn Vergaserkraftstoff in ein Dieselkraftstoff-Kraftfahrzeug eingefüllt wird. Grundsätzlich ist es aber denkbar, auch eine Zapfanlage für Dieselkraftstoff mit einer derartigen Messeinrichtung auszurüsten, z.B. um ein Bestätigungssignal zu erzeugen, dass anzeigt, dass die richtige Art von Kraftstoff getankt wird.

Hier und im folgenden wird unter "Zapfanlage" ein Zapfventil mit Zapfschlauch, zugehöriger Kraftstoffpumpe, zugeordnetem Gasrückführungssystem, zugeordneten Messgeräten, usw. verstanden, d.h. die Komponenten, die zum Tanken eines bestimmten Kraftstoffs über das gegebene Zapfventil dienen. Bei dieser Terminologie weist eine Zapfsäule in der Regel mehrere Zapfanlagen auf. Seitlich der Zapfsäule befindet sich ein Tankplatz, d.h. die Fläche, auf der ein Kraftfahrzug zum Betanken abgestellt wird, und auf der gegenüberliegenden Seite der Zapfsäule meist ein weiterer Tankplatz.

Vorzugsweise wird beim Bewerten des Messergebnisses das Messergebnis ausgewertet, und ein das Auswerteergebnis charakterisierendes Auswertesignal wird erzeugt. So kann z.B. das Messergebnis mit Sollwerten oder Sollwertbereichen verglichen werden, wobei die Sollwerte bzw. Sollwertbereiche für Dieselkraftstoff-Kraftfahrzeuge und Vergaserkraftstoff-Kraftfahrzeuge charakteristisch sind. Beim Bewerten des Messergebnisses ergibt sich also, ob es sich bei dem Einfüllstutzen des zu betankenden Kraftfahrzeugs um den eines Dieselkraftstoff-Kraftfahrzeugs oder den eines Vergaserkraftstoff-Kraftfahrzeugs handelt. Wenn hier von "Messergebnis" die Rede ist, schließt das auch die Möglichkeit ein, mehrere Einzelergebnisse auszuwerten, z.B. in Form einer Mittelung oder einer Verarbeitung der Messergebnisse mehrerer Einzelgeräte der Messeinrichtung. Auch ist es denkbar, dass zunächst Rohdaten gemessen werden, die vor dem Vergleich mit Sollwertbereichen umgewandelt oder aufbereitet werden müssen. Die für das Bewerten des Messergebnisses erforderlichen einzelnen Schritte können mit Hilfe von Hardware und/oder Software im Bereich der Messeinrichtung, des Zapfventils und/oder der zugehörigen Zapfanlage durchgeführt werden. Insbesondere ist es auch denkbar, die Zapfanlage mit entsprechenden Zusatzkomponenten zu erweitern oder einen ohnehin vorhandenen Zapfsäulenrechner mit zusätzlichen Programmen auszustatten.

Das Auswertesignal, das das Auswerteergebnis charakterisiert, kann angezeigt und/oder zum Steuern eines Betankungsvorgangs verwendet werden. Z.B. kann ein auffälliges Alarmsignal erzeugt werden, wenn bei dem Verfahren ein Dieselkraftstoff-Einfüllstutzen erfasst wird, den der Kraftfahrzeugführer gerade mit Vergaserkraftstoff betanken will. Vorzugsweise wird gleichzeitig der Betankungsvorgang blockiert.

Zum übertragen von Signalen zwischen einer Messeinrichtung im Bereich eines Zapfventils und der zugehörigen Zapfsäule kann z.B. ein Kabel dienen, das an oder im Zapfschlauch verlegt ist. Eine Nachrüstung vorhandener Anlagen wird erleichtert, wenn die Signalübertragung per Funk erfolgt.

Es kommen mehrere Größen in Frage, die für ein Dieselkraftstoff-Kraftfahrzeug charakteristisch sind und sich für eine Messung der erläuterten Art eignen. Dies sind insbesondere die Art des Kraftstoffs und die Geometrie des Einfüllstutzens.

Um die Art des Kraftstoffs zu bestimmen, kann ausgenutzt werden, dass der Dampfdruck von Dieselkraftstoff erheblich geringer ist als der von Vergaserkraftstoff. Eine Messung der Kohlenwasserstoffkonzentration im Bereich des Kraftstofftanks oder in einem aus dem Kraftstofftank abgepumpten Gas erlaubt daher eine Aussage, ob es sich um Dieselkraftstoff oder Vergaserkraftstoff und somit um ein Dieselkraftstoff-Kraftfahrzeug oder ein Vergaserkraftstoff-Kraftfahrzeug handelt.

Eine andere Möglichkeit besteht darin, die Geometrie des Einfüllstutzens zu erfassen. Wie bereits angedeutet, sind Dieselkraftstoff-Einfüllstutzen deutlich größer als die Einfüllstutzen von Vergaserkraftstoff-Kraftfahrzeugen. Eine Messung oder Abschätzung des Durchmessers oder der allgemeinen Geometrie des Einfüllstutzens erlaubt daher im Allgemeinen eine Aussage darüber, ob es sich dabei um den eines Dieselkraftstoff-Kraftfahrzeugs handelt. Im letzteren Fall sind allerdings Ausnahmen möglich. So haben Motorräder in der Regel einen Einfüllstutzen von großem Durchmesser. Auch Kanister können einen großen Deckel haben. Es gibt jedoch keine Dieselkraftstoff-Motorräder, so dass ein Betanken mit motorschädigendem Kraftstoff hierbei gar nicht möglich ist. Wenn nach dem erfindungsgemäßen Verfahren ein Motorrad oder ein Kanister betankt werden sollen, kann es also zu Fehlanzeigen kommen. Daher ist es sinnvoll, wenn das Auswertesignal den Betankungsvorgang nicht unaufhebbar blockiert, sondern wenn nach Bestätigung durch einen Benutzer der Betankungsvorgang fortgesetzt werden kann.

Um den Durchmesser des Einfüllstutzens zu erfassen, lassen sich Entfernungsmessverfahren anwenden, die z.B. mit Hilfe von Ultraschall, Mikrowellen oder Licht im sichtbaren Bereich oder im Infrarotbereich durchgeführt werden. Moderne Elektronik erlaubt Laufzeitmessungen mit einer Zeitauflösung von 1 ps, was unter Zugrundelegung der Lichtgeschwindigkeit im Vakuum einer Ortsauflösung von besser als 1 mm entspricht und damit für das Erfassen des Durchmessers eines Einfüllstutzens ausreicht.

Eine bevorzugte Möglichkeit zum Erfassen der Geometrie des Einfüllstutzens beruht auf der Bestimmung von Eigenresonanzen des Systems Zapfventil/Einfüllstutzen. Dies kann z.B. über ein Spektrum oder mit Hilfe einer Fourier-Analyse erfolgen, z.B. mit Hilfe von Ultraschall oder Mikrowellen. Die Lage der Eigenresonanzen hängt von der Geometrie eines Resonators ab, wodurch sich Unterschiede zwischen Einfüllstutzen verschiedener Geometrie ergeben. Die zugehörigen Messeinrichtungen sind bei derartigen Messungen vorzugsweise am Zapfventil angeordnet.

Die Geometrie des Einfüllstutzens kann auch durch eine Bestimmung der Verteilung von Auflagepunkten des Zapfventils an dem Einfüllstutzen bestimmt werden, vorzugsweise mit Hilfe von Drucksensoren, z.B. in Form von piezoelektrischer Folie. Wenn das Zapfventil in einen Einfüllstutzen eingeführt ist, werden je nach Größe des Einfüllstutzens die Drucksensoren an unterschiedlichen Stellen angesprochen, was einen Aufschluss über die Art des Kraftfahrzeugs erlaubt.

Bei einer weiteren Möglichkeit zum Erfassen der Geometrie des Einfüllstutzens wird zu Beginn eines Betankungsvorgangs das Gasrücktührungssystem der Zapfanlage für kurze Zeit in Betrieb genommen. Wenn dabei der mittels eines an dem Zapfventil angeordneten Sensors gemessene Druckabfall im Bereich des Einfüllstutzens relativ gering ist, ist dies ein Indiz für einen großen Einfüllstutzen, also für ein Dieselkraftstoff-Kraftfahrzeug.

Bei den bisher erläuterten Ausführungsformen werden Messungen an dem noch vorhandenen Kraftstoff oder an dem Einfüllstutzen des zu betankenden Kraftfahrzeugs durchgeführt, also Messungen im Kraftstoffbereich. Bei weiteren bevorzugten Ausführungsformen des erfindungsgemäßen Verfahrens wird als charakteristisches Merkmal die von der Zündanlage eines Vergaserkraftstoff-Kraftfahrzeugs erzeugte elektromagnetische Strahlung verwendet. Vorzugsweise wird dabei bewertet, ob vor Beginn eines Betankungsvorgangs, der an einer vorgegebenen Zapfanlage erfolgen soll, eine von dem zu betankenden Kraftfahrzeug ausgehende elektromagnetische Strahlung gemessen worden ist. Wurde eine solche vom Funkenüberschlag in Zündkerzen erzeugte Störstrahlung gemessen, handelt es sich um ein Vergaserkraftstoff-Kraftfahrzeug. Wurde dagegen keine derartige elektromagnetische Strahlung gemessen (oder liegt ein gemessenes Signal unter einer vorgegebenen Schwelle), handelt es sich mit hoher Wahrscheinlichkeit um ein Dieselkraftstoff-Kraftfahrzeug, das auf keinen Fall mit Vergaserkraftstoff betankt werden darf.

Die von Zündanlagen von Vergaserkraftstoff-Kraftfahrzeugen erzeugte elektromagnetische Strahlung kann mit einer auf der Tankstelle angeordneten Detektoranlage, die vorzugsweise mindestens eine Antenne aufweist, überwacht und räumlich den zu jeweiligen Zapfanlagen vorfahrenden Vergaserkraftstoff-Kraftfahrzeugen zugeordnet werden; bei einer vorgegebenen Zapfanlage wird bewertet, ob vor Beginn eines Betankungsvorgangs dem zu betankenden Kraftfahrzeug elektromagnetische Strahlung zugeordnet worden ist.

Vorzugsweise wird beim Bewerten ein Signal verwendet, das für das Vorhandensein eines Kraftfahrzeugs im Bereich einer vorgegebenen Zapfanlage charakteristisch ist. Ein derartiges Signal kann z.B. mittels mindestens einer Induktionsschleife im Bereich der Zapfanlage erzeugt werden. Die Ausnutzung dieses Signals vereinfacht die Bewertung (Auswertung) und erhöht deren Zuverlässigkeit, denn es zeigt an, ob sich gerade ein Kraftfahrzeug an der Zapfanlage befindet, unabhängig von den Signalen der auf die elektromagnetische Strahlung ansprechenden Detektoranlage. So ist es z.B. denkbar, auf eine aufwändige räumliche und zeitliche Auswertung der Bewegung der Kraftfahrzeuge auf der Tankstelle zu verzichten und statt dessen an jeder Zapfsäule eine Antenne und an jedem Tankplatz eine Induktionsschleife anzuordnen. Wenn die Antenne nach dem Ansprechen der Induktionsschleife für kurze Zeit (d.h. vor dem Ausschalten des Motors des gerade vorgefahrenen Kraftfahrzeugs) relativ starke elektromagnetische Störstrahlung misst, ist an diesem Tankplatz gerade ein Vergaserkraftstoff-Kraftfahrzeug angekommen. Tritt dagegen keine Störstrahlung auf, ist das von der Induktionsschleife erfasste Kraftfahrzeug ein Dieselkraftstoff-Kraftfahrzeug.

Auch bei der Verwendung der von Zündanlagen von Vergaserkraftstoff-Kraftfahrzeugen erzeugten elektromagnetischen Strahlung können die zum Weiterverarbeiten, Auswerten und Bewerten der einzelnen Messwerte und Messergebnisse erforderlichen Schritte in Hardware-Einrichtungen, Prozessoren und Rechnern durchgeführt werden, z.B. im Bereich der Zapfanlagen der Tankstelle, ähnlich wie weiter oben im Zusammenhang mit den Messungen im Kraftstoffbereich erläutert. Vorzugsweise wird ein Alarmsignal abgegeben und/oder der Tankvorgang blockiert, wenn versucht wird, ein Dieselkraftstoff-Kraftfahrzeug mit Vergaserkraftstoff zu betanken.

Im Folgenden wird die Erfindung anhand von Ausführungsbeispielen weiter erläutert. Die Zeichnungen zeigen in
- Fig. 1: eine schematische Ansicht eines Betankungsvorgangs auf einer Tankstelle und
- Fig. 2: eine schematische Ansicht eines in einen Einfüllstutzen eines Kraftfahrzeugs eingeführten Zapfventils mit einer daran angeordneten Messeinrichtung.

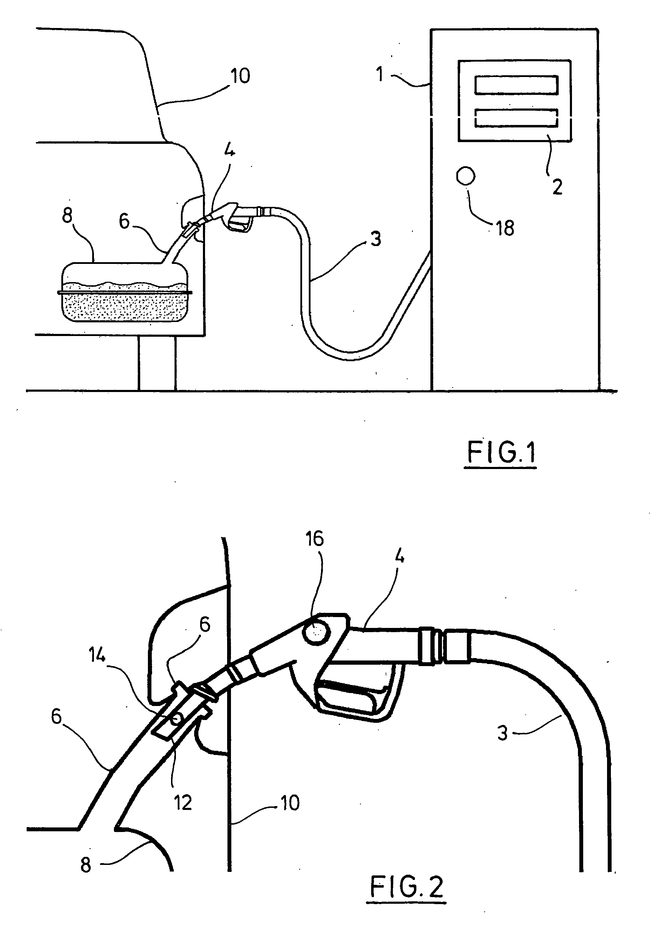

In Fig. 1 ist schematisch veranschaulicht, wie ein Kraftfahrzeug an einer Tankstelle betankt wird. Von einer Zapfsäule 1 mit einer Anzeige 2 geht ein Zapfschlauch 3 mit einem Zapfventil 4 aus. Das Zapfventil 4 ist in einen Einfüllstutzen 6 eines Kraftstofftanks 8 des mit 10 bezeichneten Kraftfahrzeugs eingeführt.

Fig. 2 zeigt in vergrößerter Ansicht, wie sich der vordere Bereich 12 des Zapfventils 4 im Innenraum des Einfüllstutzens 6 befindet. Am vorderen Bereich 12 des Zapfventils 4 ist eine Messeinrichtung 14 angeordnet, wozu im Folgenden einige Beispiele angegeben werden. Die Messeinrichtung ist bei der Ausführungsform gemäß Fig. 2 elektrisch mit einer Elektronikeinheit 16 verbunden, die sich im mittleren oder hinteren Bereich des Zapfventils 4 befindet. Die Elektronikeinheit 16 kann zusätzliche Elektronikbausteine enthalten, die für den Betrieb der Messeinrichtung 14 erforderlich sind, und weist in der gezeigten Ausführungsform außerdem einen Sender auf, um die von der Messeinrichtung 14 und der Elektronikeinheit 16 erhaltenen Mess- bzw. Auswerteergebnisse zu der Zapfsäule 1 zu übermitteln. In der Zapfsäule 1 befindet sich ein auf den Sender abgestimmter Empfänger. In der Zapfsäule 1 ist eine weitere Verarbeitung der übermittelten Mess- bzw. Auswerteergebnisse möglich, z.B. in Elektronikbausteinen oder in dem vorhandenen Zapfsäulenrechner.

Wenn der in der Elektronikeinheit 16 vorhandene Sender zur Datenübermittlung verwendet wird, ist eine Nachrüstung einer vorhandenen Zapfanlage besonders einfach, denn in diesem Fall braucht der Zapfschlauch 3 nicht verändert zu werden, um darin Datenkabel zu verlegen.

Mit Hilfe der bei der Auswertung erhaltenen Signale lässt sich ein Betankungsvorgang steuern, und das Ergebnis kann auch auf der Anzeige 2 der Zapfsäule 1 angezeigt werden. Im Ausführungsbeispiel ist die Zapfsäule 1 zum Tanken von Vergaserkraftstoff eingerichtet.

Wenn über die Messeinrichtung 14 erkannt wird, dass der Einfüllstutzen 6 zu einem Kraftstofftank 8 gehört, der bereits Dieselkraftstoff enthält, wird ein Betankungsvorgang vorzugsweise sofort abgebrochen oder von vornherein blockiert. Bei anderen Messverfahren wird die Geometrie des Einfüllstutzens 6 erfasst, und wenn sich dabei ergibt, dass es sich um einen großen Einfüllstutzen handelt, wird vorzugsweise ebenfalls ein Betankungsvorgang abgebrochen oder blockiert. Dies wird auf der Anzeige 2 angezeigt. Da es auch große Einfüllstutzen gibt, die nicht zu Dieselkraftstoff-Kraftfahrzeugen gehören, wie eingangs erläutert, ist es in diesem Fall sinnvoll, wenn der Benutzer nach ausdrücklicher Bestätigung (z.B. durch Drücken auf einen Knopf 18 an der Zapfsäule 1, siehe Figur 1) den Betankungsvorgang trotzdem durchführen kann.

Wie bereits angedeutet, kann die Messeinrichtung 14 einen Sensor enthalten, mit dem sich die Kohlenwasserstoffkonzentration bestimmen lässt. Dies ist z.B. mit Hilfe einer Wärmeleitfähigkeitsmesszelle möglich. Wenn die Kohlenwasserstoffkonzentration niedrig ist, befindet sich in dem Kraftstofftank 8 Dieselkraftstoff, denn der Dampfdruck von Dieselkraftstoff ist erheblich niedriger als der von Vergaserkraftstoff.

Bei einer anderen Möglichkeit, die Art des Kraftstoffs festzustellen, wird eine Messeinrichtung verwendet, die sich nicht am Zapfventil 4 befindet, sondern ohnehin in einer modernen Zapfsäule 1 enthalten ist. Dabei wird ein Betankungsvorgang für eine kurze Zeit (z.B. 2 Sekunden) gestartet, und die in dem Gasrückführungssystem der für Vergaserkraftstoff bestimmten Zapfsäule 1 vorhandene Gasrückführungspumpe fördert Gas aus dem Kraftstofftank 8. Moderne Gasrückführungssysteme enthalten eine Einrichtung zum Messen der Kohlenwasserstoffkonzentration, die in ähnlicher Weise wie der zuvor erläuterte Sensor dazu benutzt werden kann, die Art des Kraftstoffs zu erfassen. Wenn sich dabei herausstellt, dass es sich um Dieselkraftstoff handelt, wird der Betankungsvorgang abgebrochen. Innerhalb der kurzen Zeitspanne von z.B. 2 Sekunden kann nicht so viel Vergaserkraftstoff in den Krafststofftank 8 gelangen, dass es zu bleibenden Schäden an einem Dieselkraftstoff-Kraftfahrzeug kommt.

In weiteren Ausführungsformen enthält die Messeinrichtung 14 mit der zugeordneten Elektronikeinheit 16 eine Einrichtung zur Entfernungsmessung, um den Abstand zwischen der momentanen Lage der Messeinrichtung 14 und der Wandung des Einfüllstutzens 6 zu bestimmen. In den Ausführungsbeispielen wird die Entfernungsmessung über die Laufzeit von Ultraschall-, Mikrowellen- oder optischen Impulsen durchgeführt. Dazu werden Impulse von der Messeinrichtung 14 in Richtung auf die Wandung des Einfüllstutzens 6 ausgesandt, und die Laufzeit des anschließend empfangenen reflektierten Signals wird gemessen. Da die Laufzeit des Signals zumindest weitgehend bekannt ist, lässt sich daraus der Abstand zwischen der Messeinrichtung 14 und der Wandung des Einfüllstutzens 6 ermitteln. Vorzugsweise werden mehrere Einzelmessungen durchgeführt, auch unter Aussendung der Impulse in verschiedene Richtungen, und als Auswerteergebnis wird eine Mittelung der aufbereiteten Messergebnisse verwendet. Wenn die mittlere Laufzeit groß ist, handelt es sich um einen großen Einfüllstutzen 6, also wahrscheinlich um ein Dieselkraftstoff-Kraftfahrzeug.

Bei metallischen Einfüllstutzen 6 ist auch die Kapazität des aus dem Zapfventil 4 und dem Einfüllstutzen 6 bestehenden Systems ein Maß für die Größe des Einfüllstutzens 6. Um dies für ein Messverfahren zu nutzen, enthält die Messeinrichtung 14 eine Kapazitätsmesseinrichtung.

Bei anderen Ausführungsformen wird die Geometrie des Einfüllstutzens 6 mit Hilfe von Resonanzmessungen erfasst.

Dazu werden z.B. über die Messeinrichtung 14 Ultraschallwellen ausgesendet, deren Frequenz durchgestimmt wird. Bei Resonanzfrequenzen tritt eine hohe Schallamplitude auf, was über die Messeinrichtung 14 erfasst wird. Die Lage der Resonanzfrequenzen ist ein Maß für die Geometrie des Einfüllstutzens 6. Niedrige Resonanzfrequenzen deuten auf einen großen Einfüllstutzen hin, also ein Dieselkraftstoff-Kraftfahrzeug. Derartige Ultraschallmessungen führen auch zu einem Ergebnis, wenn der Einfüllstutzen 6 nicht aus Metall besteht.

Vergleichbare Resonanzmessungen lassen sich ebenfalls mit Hilfe von Mikrowellen durchführen. Die Frequenzanalyse kann generell auch über Fourier-Techniken erfolgen.

Bei anderen Ausführungsformen weist die Messeinrichtung 14 eine Anzahl von Drucksensoren auf, die auf mechanischen Druck ansprechen und am vorderen Bereich 12 des Zapfventils 4 verteilt sind. Dazu kann z.B. eine größere piezoelektrische Folie verwendet werden, die Signale mit Ortsauflösung abgibt. Wenn das Zapfventil 4 in den Einfüllstutzen 6 eingeführt ist, drückt die Wandung des Einfüllstutzens 6 auf bestimmte Stellen an dem Zapfventil 4, und zwar in Abhängigkeit davon, wie groß der Einfüllstutzen 6 ist und wo die Krümmung seiner Wandung verläuft. Wenn die Druckstellen von den Drucksensoren erfasst werden, lässt sich mittels einer Analyse der Verteilung der Druckstellen bestimmen, ob der Einfüllstutzen 6 groß ist oder klein.

Bei einer weiteren Ausführungsform enthält die Messeinrichtung 14 einen Drucksensor, der auf einen Gasdruck anspricht. Vor Beginn eines Betankungsvorgangs befindet sich dieser Drucksensor auf Atmosphärendruck. Wenn der Betankungsvorgang gestartet wird, fördert das Gasrückführungssystem das Gas über dem Kraftstoff in dem Kraftstofftank 8 in Richtung Zapfsäule 1, so dass es innerhalb des ringförmigen Raums zwischen der Wandung des Einfüllstutzens 6 und dem vorderen Bereich 12 des Zapfventils 4 zu einem Druckabfall kommt; am oberen Ende des Einfüllstutzens 6 herrscht nach wie vor Atmosphärendruck, während an der Stelle des Drucksensors 14 in dem erwähnten ringförmigen Raum der Gasdruck etwas höher ist, was von dem Drucksensor 14 erfasst wird. Die Druckdifferenz ist ein Maß für den Strömungswiderstand in dem ringförmigen Raum. Wenn der Strömungswiderstand gering ist, handelt es sich wahrscheinlich um einen großen Einfüllstutzen 6. In diesem Fall wird der Betankungsvorgang sofort abgebrochen, ähnlich wie in einem der zuvor erläuterten Ausführungsbeispiele.

Bei weiteren Ausführungsformen wird ausgenutzt, dass Kraftfahrzeuge mit Vergaserkraftstoffbetrieb mit einer Hochspannungszündung ausgerüstet sind. Der Funkenüberschlag in der Zündkerze erzeugt ein großes Spektrum an elektromagnetischen Wellen, die zum Teil abgestrahlt werden und als Störungen auftreten. Diese charakteristische pulsierende Störung tritt bei einem Kraftfahrzeug mit Dieselmotor nicht auf.

Um die Art des Kraftfahrzeuges zu erkennen, werden Hochfrequenzempfänger geeignet auf der Tankstelle plaziert, um die Annäherung und Tankposition von Vergaserkraftstoff-Kraftfahrzeugen zu erfassen. Wird bei einem Tankplatz ein Vergaserkraftstoff-Zapfventil abgehoben und es wurde zuvor keine Annäherung eines Vergaserkraftstoff-Kraftfahrzeuges detektiert, so wird eine Warnungsmeldung erzeugt.

Die Empfänger bzw. die Antennen können z.B. in den Zapfsäulen, in der Fahrbahn oder auch an dem Stationsdach angebracht sein. Durch das Zusammenführen der Messdaten von allen Messpunkten kann sichergestellt werden, dass eine korrekte Erkennung der Positionen der auf der Tankstelle vorhandenen Vergaserkraftstoff-Kraftfahrzeuge erreicht wird. Hierzu muss auch der zeitliche Verlauf der Daten bewertet werden, da die Fahrzeuge vor der Betankung ausgeschaltet werden. Es wird dadurch jede Annäherung, jeder Halt und jede Wiederentfernung von Kraftfahrzeugen mit Ottomotor registriert.

Zusätzlich zu dem Antennensystem können zur Identifikation eines Kraftfahrzeuges an jedem Tankplatz z.B. im Boden oder auch in der Zapfsäule Induktionsschleifen eingelassen sein. Mit den darüber erzeugten Signalen können jede Annäherung und das Vorhandensein von Kraftfahrzeugen (Metallmasse) erkannt werden. Zusammen mit der Auswertung der Information von den Antennen bezüglich Vergaserkraftstoff-Kraftfahrzeugen können diese und die zugehörigen Tankplatze bestimmt werden. Bei besetzten Tankplätzen, wo zuvor keine Antennensignale (Störimpulse der Zündung) erkannt wurden, muss es sich folglich um ein Dieselkraftstoff-Kraftfahrzeug handeln. Erfolgt auf einem solchen Tankplatz das Abheben des Vergaserkraftstoff-Zapfventils, wird eine Alarmmeldung abgegeben.

## Patentansprüche

1. Verfahren zum Erfassen der Kraftstoffart von Kraftfahrzeugen an einer Tankstelle, **dadurch gekennzeichnet, dass** vor Beginn und/oder zu Beginn eines Betankungsvorgangs ein für ein Dieselkraftstoff-Kraftfahrzeug und/oder ein Vergaserkraftstoff-Kraftfahrzeug charakteristisches Merkmal gemessen und bewertet wird.

2. Verfahren nach Anspruch 1, **gekennzeichnet durch** die Schritte:
- Durchführen einer Messung einer für ein Dieselkraftstoff-Kraftfahrzeug (10) charakteristischen Größe mittels einer im Bereich eines Zapfventils (4) und/oder einer zugehörigen Zapfanlage (1, 3), vorzugsweise für Vergaserkraftstoff, angeordneten Messeinrichtung (14), wobei die Messung vorzugsweise durchgeführt wird, wenn das Zapfventil (4) in einen Einfüllstutzen (6) eingeführt ist,
- Bewerten des Messergebnisses.

3. Verfahren nach Anspruch 2, **dadurch gekennzeichnet, dass** beim Bewerten des Messergebnisses das Messergebnis ausgewertet wird und ein das Auswerteergebnis charakterisierendes Auswertesignal erzeugt wird.

4. Verfahren nach Anspruch 3, **dadurch gekennzeichnet**, das das Auswertesignal angezeigt wird und/oder zum Steuern eines Betankungsvorgangs verwendet wird.

5. Verfahren nach einem der Ansprüche 2 bis 4, **dadurch gekennzeichnet, dass** die für ein Dieselkraftstoff-Kraftfahrzeug (10) charakteristische Größe die Art des Kraftstoffs ist.

6. Verfahren nach Anspruch 5, **dadurch gekennzeichnet, dass** zu Beginn eines Betankungsvorgangs die Kohlenwasserstoffkonzentration im Bereich des Kraftstofftanks (8) des zu betankenden Kraftfahrzeugs (10) über dem flüssigen Kraftstoff mit einem an dem Zapfventil (4) angeordneten Sensor (14) gemessen wird.

7. Verfahren nach Anspruch 5, **dadurch gekennzeichnet, dass** zu Beginn eines Betankungsvorgangs eine Gasrückführungspumpe der Zapfanlage (1, 3) für eine vorgegebene Zeit in Betrieb genommen und die Kohlenwasserstoffkonzentration des aus dem Kraftstofftank (8) des zu betankenden Kraftfahrzeugs (10) geförderten Gases gemessen wird.

8. Verfahren nach einem der Ansprüche 2 bis 4, **dadurch gekennzeichnet, dass** die für ein Dieselkraftstoff-Kraftfahrzeug (10) charakteristische Größe die Geometrie des Einfüllstutzens (6) ist.

9. Verfahren nach Anspruch 8, **dadurch gekennzeichnet, dass** die für ein Dieselkraftstoff-Kraftfahrzeug (10) charakteristische Größe der Durchmesser des Einfüllstutzens (6) ist.

10. Verfahren nach Anspruch 9, **dadurch gekennzeichnet, dass** der Durchmesser des Einfüllstutzens (6) mittels einer Ultraschall-Entfernungsmessung erfasst wird.

11. Verfahren nach Anspruch 9, **dadurch gekennzeichnet, dass** der Durchmesser des Einfüllstutzens (6) mittels einer Mikrowellen-Entfernungsmessung erfasst wird.

12. Verfahren nach Anspruch 9, **dadurch gekennzeichnet, dass** der Durchmesser des Einfüllstutzens (6) mittels einer optischen Entfernungsmessung erfasst wird.

13. Verfahren nach Anspruch 9, **dadurch gekennzeichnet, dass** der Durchmesser des Einfüllstutzens (6) mittels einer Kapazitätsmessung erfasst wird.

14. Verfahren nach Anspruch 8, **dadurch gekennzeichnet, dass** zum Erfassen der Geometrie des Einfüllstutzens (6) mittels Ultraschallmessungen Eigenresonanzen des den Einfüllstutzen (6) und das Zapfventil (4) aufweisenden Systems bestimmt werden.

15. Verfahren nach Anspruch 8, **dadurch gekennzeichnet, dass** zum Erfassen der Geometrie des Einfüllstutzens (6) mittels Mikrowellenmessungen Eigenresonanzen des den Einfüllstutzen (6) und das Zapfventil (4) aufweisenden Systems bestimmt werden.

16. Verfahren nach Anspruch 8, **dadurch gekennzeichnet, dass** zum Erfassen der Geometrie des Einfüllstutzens (6) eine Verteilung von Auflagepunkten des Zapfventils (4) an dem Einfüllstutzen (6) bestimmt wird.

17. Verfahren nach Anspruch 16, **dadurch gekennzeichnet, dass** die Verteilung von Auflagepunkten mittels Drucksensoren (14), vorzugsweise in Form von piezoelektrischer Folie, an dem Zapfventil (4) bestimmt wird.

18. Verfahren nach Anspruch 8, **dadurch gekennzeichnet, dass** zum Erfassen der Geometrie des Einfüllstutzens (6) zu Beginn eines Betankungsvorgangs eine Gasrückführungspumpe der Zapfanlage (1, 3) für eine vorgegebene Zeit in Betrieb genommen und der Druckabfall im Bereich des Einfüllstutzens (6) mittels eines an dem Zapfventil (4) angeordneten Sensors (14) gemessen wird.

19. Verfahren nach Anspruch 1, **dadurch gekennzeichnet, dass** das charakteristische Merkmal die von der Zündanlage eines Vergaserkraftstoff-Kraftfahrzeugs erzeugte elektromagnetische Strahlung ist.

20. Verfahren nach Anspruch 19, **dadurch gekennzeichnet, dass** bewertet wird, ob vor Beginn eines an einer vorgegebenen Zapfanlage zu erfolgenden Betankungsvorgangs von dem zu betankenden Kraftfahrzeug ausgehende elektromagnetische Strahlung gemessen worden ist.

21. Verfahren nach Anspruch 19 oder 20, **dadurch gekennzeichnet, dass** die von Zündanlagen von Vergaserkraftstoff-Kraftfahrzeugen erzeugte elektromagnetische Strahlung mit einer auf der Tankstelle angeordneten Detektoranlage, die vorzugsweise mindestens eine Antenne aufweist, überwacht und räumlich den zu jeweiligen Zapfanlagen vorfahrenden Vergaserkraftstoff-Kraftfahrzeugen zugeordnet wird und dass bei einer vorgegebenen Zapfanlage bewertet wird, ob vor Beginn eines Betankungsvorgangs dem zu betankenden Kraftfahrzeug elektromagnetische Strahlung zugeordnet worden ist.

22. Verfahren nach einem der Ansprüche 19 bis 21, **dadurch gekennzeichnet, dass** beim Bewerten ein Signal verwendet wird, das für das Vorhandensein eines Kraftfahrzeugs im Bereich einer vorgegebenen Zapfanlage charakteristisch ist.

23. Verfahren nach Anspruch 22, **dadurch gekennzeichnet, dass** das Signal mittels mindestens einer Induktionsschleife im Bereich der Zapfanlage erzeugt wird.

24. Vorrichtung zur Durchführung eines Verfahrens nach einem der Ansprüche 1 bis 23.

25. Vorrichtung nach Anspruch 24, mit einer im Bereich eines Zapfventils (4) und/oder einer zugehörigen Zapfanlage (1, 3), vorzugsweise für Vergaserkraftstoff, angeordneten oder montierbaren Messeinrichtung (14), die zum Durchführen einer Messung einer für ein Dieselkraftstoff-Kraftfahrzeug (10) charakteristischen Größe eingerichtet ist, und mit einer zum Bewerten des Messergebnisses eingerichteten Auswerteeinrichtung.

26. Vorrichtung nach Anspruch 25, **dadurch gekennzeichnet, dass** die Messeinrichtung (14) mindestens eine der aus der folgenden Liste ausgewählten Einrichtungen aufweist: an dem Zapfventil (4) angeordneter oder montierbarer Sensor (14) zum Messen einer Kohlenwasserstoffkonzentration, am Gasrückführüngssystem der Zapfanlage (1, 3) angeordneter oder montierbarer Gassensor, im Bereich des Zapfventils (4) angeordenete oder montierbare Ultraschall-Entfernungsmesseinrichtung (14), im Bereich des Zapfventils (4) angeordenete oder montierbare Mikrowellen-Entfernungsmesseinrichtung (14), im Bereich des Zapfventils (4) angeordenete oder montierbare optische Entfernungsmesseinrichtung (14), im Bereich des Zapfventils (4) angeordenete oder montierbare Kapazitätsmesseinrichtung (14), im Bereich des Zapfventils (4) angeordenete oder montierbare Ultraschall-Resonanzmesseinrichtung (14), im Bereich des Zapfventils (4) angeordenete oder montierbare Mikrowellen-Resonanzmesseinrichtung (14), im Bereich des Zapfventils (4) angeordenete oder montierbare Drucksensoren (14), im Bereich des Zapfventils (4) angeordeneter oder montierbarer Gasdrucksensor (14).

27. Vorrichtung nach Anspruch 24, mit einer Detektoranlage und einer zugeordneten Auswerte- und Bewertungseinrichtung, die zum Messen und räumlichen Zuordnen der von Zündanlagen von Vergaserkraftstoff-Kraftfahrzeugen erzeugten elektromagnetischen Strahlung eingerichtet sind.
